# EUROPEAN PATENT APPLICATION

(11) **EP 4 782 554 A1**
(43) Date of publication of application: **29.07.2026**
(21) Application number: 23953195.7
(22) Date of filing: 03.11.2023
(51) Int. Cl.: C12Q 1/6886, G01N 33/574

(54) **COMPOSITION FOR DIAGNOSING RADIOSENSITIVITY OF BREAST CANCER OR PROGNOSING BREAST CANCER**

(30) Priority: 20.09.2023 KR 20230125814
(71) Applicant: Korea Institute of Radiological & Medical Sciences, Seoul 01812 (KR)
(72) Inventor: SONG, Jie Young, Seoul 07324 (KR); PARK, Jeong In, Seoul 02562 (KR); SONG, Kyung Hee, Namyangju-si, Gyeonggi-do 12141 (KR); LEE, Dong Hyeon, Gunpo-si, Gyeonggi-do 15810 (KR); JUNG, Seung Youn, Seoul 01817 (KR); LEE, Jee Yong, Seoul 06194 (KR); AHN, Jiyeon, Seoul 01721 (KR); PARK, Jong Kuk, Seoul 05262 (KR); HWANG, Sang Gu, Seoul 01727 (KR)
(74) Representative: Cabinet Beau de Loménie
(86) International application number: PCT/KR2023/017589
(87) International publication number: WO 2025/063372

(57) **Abstract**

In the present invention, the mRNA of genes CD68, IFI16, TNFR2, CXCL5, LCN2, SERPING1, MATN1, and SYTL4, or the proteins encoded by these genes, exhibit differential expression levels in radioresistant and radiosensitive breast cancer cells and have a close correlation with the increased survival rate of breast cancer patients. Therefore, an agent for measuring the expression levels of mRNA or proteins encoded by genes selected from the group consisting of CD68, IFI16, TNFR2, CXCL5, LCN2, SERPING1, MATN1, and SYTL4 of the present invention can effectively diagnose the radiosensitivity of breast cancer or prognose breast cancer radiotherapy. By determining the necessity of radiotherapy and optimizing the radiation dose, the agent can enhance the efficiency and outcomes of breast cancer radiotherapy.

## Description

### [Technical Field]

### CROSS-REFERENCE TO RELATED APPLICATION

This application claims the benefit of priority to Korean Patent Application No. 10-2023-0125814, filed in the Korean Intellectual Property Office on September 20, 2023, the entire contents of which are incorporated herein by reference.

### [Technical Field]

The present disclosure relates to a composition for diagnosing radiosensitivity of breast cancer and an application thereof.

### [Background Art]

Cancer treatment methods include surgical operation, chemical drug therapy, and radiotherapy, and recently, the importance of radiotherapy has been further increasing. Cases, in which tumors have been efficiently cured only by radiotherapy when surgical operation is difficult, have been reported, and tumor treatment methods using radiation have also been developing year by year, and accordingly, radiotherapy has been established as a method capable of efficiently treating tumors in the body without causing special pain or resistance to patients. Statistically, about 30-50% of cancer patients receive radiotherapy at some point during their treatment period. Because the number of cancer patients who receive radiotherapy has been increasing every year, the importance of radiotherapy in cancer treatment is also increasing.

Radiotherapy is commonly used either alone or in combination with chemotherapeutic agents to treat various types of cancer. However, the effectiveness of radiotherapy varies depending on the characteristics of the cancer, the patient, and whether the radiation is combined with other treatments. In general, cancers, on which radiotherapy is widely performed, include cervical cancer, pharyngeal cancer, lung cancer, brain cancer, and breast cancer. Although these types of cancer are major targets of radiotherapy, poor responses to radiotherapy frequently occur, and thus, strategies to improve treatment efficiency are required. In addition, although radiotherapy may be well performed and show a good initial response, recurrence frequently occurs, and thus, establishing countermeasures against recurrent cancer is also important for improving the efficiency of radiotherapy.

Furthermore, acquisition of radioresistance by cancer cells and damage to normal tissues during high-dose radiotherapy have been pointed out as problems that reduce the efficiency of radiotherapy, and thus, research to enhance the efficiency of radiotherapy is required. Currently, radiotherapy, together with surgical operation or chemical drug therapy, is an effective cancer treatment method and is used for the purpose of killing cancer cells by applying radiation to the cancer tissue of a patient.

Although there are differences in the sensitivity of cancer cells to radiotherapy, by developing a technique capable of predicting individual sensitivity to radiation before radiotherapy, it would be possible to treat cancer by adjusting the radiation dose for each individual, thereby providing the most suitable radiotherapy for each patient.

Meanwhile, in recent years, studies on companion diagnostics, which refer to approved diagnostics that may select appropriate targeted anticancer drugs and treatment methods based on systematic analysis results of individual patient factors, have been actively conducted. Because companion diagnostics may provide clear clinical evidence for prescriptions based on a physician's diagnosis and suggest appropriate treatment methods for patients, they may not only improve the efficiency of cancer treatment but also reduce the misuse or overuse of targeted anticancer drugs, thereby contributing to the financial soundness of the national health insurance system. Currently, the companion diagnostics market is growing in therapeutic fields such as breast cancer, lung cancer, colorectal cancer, gastric cancer, and melanoma, and in particular, the fields of breast cancer and lung cancer are expected to drive market growth.

Korean Patent Application No. 10-2018-0143493 discloses a technology of measuring the expression level of a PMVK protein or mRNA to use it for diagnosing radiation-resistant lung cancer or pancreatic cancer, but does not provide any data regarding breast cancer cell specificity.

Furthermore, Korean Patent Application No. 10-2018-0153659 discloses a technology of diagnosing radioresistance of cancer cells by measuring the expression level of a ROMO1 gene or protein, but does not suggest a technology capable of more accurately diagnosing radiosensitive breast cancer by using multiple markers.

Under this background, the inventors of the present disclosure conducted research on radiosensitivity biomarkers and discovered novel biomarkers for diagnosing radiosensitive breast cancer. The biomarkers are expected to be usefully applicable to breast cancer treatment, because they may predict sensitivity to radiotherapy or the prognosis of radiotherapy.

### [Prior art documents]

### [Patent documents]

Korean Patent Application No. 10-2018-0143493
Korean Patent Application No. 10-2018-0153659

### [Disclosure]

### [Technical Problem]

An aspect of the present disclosure provides a composition for diagnosing radiosensitivity of breast cancer.

An aspect of the present disclosure also provides a kit for diagnosing radiosensitivity of breast cancer.

An aspect of the present disclosure also provides a method for providing information for diagnosing radiosensitivity of breast cancer.

An aspect of the present disclosure also provides a composition for predicting a prognosis of breast cancer radiotherapy.

An aspect of the present disclosure also provides a kit for predicting a prognosis of breast cancer radiotherapy.

An aspect of the present disclosure also provides a method for providing information for predicting a prognosis of breast cancer radiotherapy.

### [Technical Solution]

An aspect of the present disclosure provides a composition for diagnosing radiosensitivity of breast cancer, comprising an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

Furthermore, another aspect of the present disclosure provides a kit for diagnosing radiosensitivity of breast cancer, comprising the composition for diagnosing radiosensitivity of breast cancer.

Furthermore, another aspect of the present disclosure provides a method for providing information for diagnosing radiosensitivity of breast cancer, comprising (a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes, and (b) comparing the expression levels of the mRNA or proteins with expression levels of mRNA or proteins of a normal control group.

In addition, another aspect of the present disclosure provides a composition for predicting a prognosis of breast cancer radiotherapy, the composition comprising an agent for measuring expression levels of mRNA of two or more genes selected from the group consisting of CD68, IFI16, TNFR2, CXCL5, LCN2, SERPING1, MATN1, and SYTL4, or proteins encoded by the genes.

Furthermore, another aspect of the present disclosure provides a kit for predicting a prognosis of breast cancer radiotherapy, which comprises the composition for predicting a prognosis of breast cancer radiotherapy.

Furthermore, another aspect of the present disclosure provides a method for providing information for predicting a prognosis of breast cancer radiotherapy, comprising (a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes, and (b) comparing the expression levels of the mRNA or proteins with expression levels of mRNA or proteins of a normal control group.

Hereinafter, the present disclosure will be described in detail.

### 1. Composition for Diagnosing Radiosensitivity of Breast Cancer or Predicting Prognosis of Breast Cancer Radiotherapy

An aspect of the present disclosure provides a composition for diagnosing radiosensitivity of breast cancer.

Another aspect of the present disclosure provides a composition for predicting a prognosis of breast cancer radiotherapy.

The composition for diagnosing radiosensitivity of breast cancer according to the present disclosure includes, as an active ingredient, an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

In addition, the composition for predicting a prognosis of breast cancer radiotherapy according to the present disclosure includes, as an active ingredient, an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

The cluster of differentiation 68 (CD68) (NCBI Gene ID: 968) protein is a protein expressed in cells of the monocyte lineage and in macrophages, and is generally known as a scavenger receptor that removes cellular debris, promotes phagocytosis, and mediates the recruitment and activation of macrophages.

In the present disclosure, the CD68 protein refers to not only the wild-type CD68 protein consisting of the amino acid sequence of SEQ ID No: 1, but also equivalents having the same or similar function or activity as the wild-type CD68 protein, although their sequences are partially different, and, the equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 1.

The interferon gamma inducible protein 16 (IFI16) (NCBI Gene ID: 3428) protein is known as an interferon-inducible myeloid differentiation transcriptional activator that interacts with p53, retinoblastoma-1 (RB1), and BRCA1, and functions in cell growth inhibition and intracellular DNA sensing.

In the present disclosure, the IFI16 protein refers to not only the wild-type IFI16 protein consisting of the amino acid sequence of SEQ ID No: 2, but also equivalents having the same or similar function or activity as the wild-type IFI16 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 2.

The tumor necrosis factor receptor 2 (TNFR2) (NCBI Gene ID: 7133) protein, also referred to as tumor necrosis factor receptor superfamily member 1B (TNFRSF1B), is one of the membrane receptors that bind to TNFα, and is known to induce transcription of genes related to cell survival, growth, and differentiation.

In the present disclosure, the TNFR2 protein refers to not only the wild-type TNFR2 protein consisting of the amino acid sequence of SEQ ID No: 3, but also equivalents having the same or similar function or activity as the wild-type TNFR2 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 3.

CXCL5 (C-X-C motif chemokine ligand 5, NCBI gene ID: 6374) is a small cytokine belonging to the CXC chemokine family, and it is known to be produced in response to inflammatory cytokines interleukin-1 and tumor necrosis factor-alpha, and suppress type II interferon-gamma to regulate neutrophil homeostasis.

In the present disclosure, the CXCL5 protein refers to not only the wild-type CXCL5 protein consisting of the amino acid sequence of SEQ ID No: 4, but also equivalents having the same or similar function or activity as the wild-type CXCL5 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 4.

The lipocalin 2 (LCN2) (NCBI Gene ID: 3934) protein, also referred to as neutrophil gelatinase-associated lipocalin (NGAL), is known to be involved in innate immunity by sequestering iron and preventing its utilization by bacteria, thereby restricting their growth.

In the present disclosure, the LCN2 protein refers to not only the wild-type LCN2 protein consisting of the amino acid sequence of SEQ ID No: 5, but also equivalents having the same or similar function or activity as the wild-type LCN2 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 5.

The serpin family G member 1 (SERPING1) (NCBI Gene ID: 710) protein is a protease inhibitor belonging to the serpin superfamily. It is also referred to as the C1 esterase inhibitor (C1-INH) and is known to regulate physiological pathways, including complement activation, blood coagulation, fibrinolysis, and kinin generation, by inhibiting the activation of the C1 complex.

In the present disclosure, the SERPING1 protein refers to not only the wild-type SERPING1 protein consisting of the amino acid sequence of SEQ ID No: 6, but also equivalents having the same or similar function or activity as the wild-type SERPING1 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 6.

The matrilin 1 (MATN1) (NCBI Gene ID: 4146) protein is a cartilage matrix protein involved in the formation of filament networks in the extracellular matrix of various tissues, and mutations in this gene are known to be associated with various hereditary chondrodysplasias.

In the present disclosure, the MATN1 protein refers to not only the wild-type MATN1 protein consisting of the amino acid sequence of SEQ ID No: 7, but also equivalents having the same or similar function or activity as the wild-type MATN1 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 7.

The synaptotagmin like 4 (SYTL4) (NCBI Gene ID: 94121) protein has an N-terminal Rab27-binding domain and a C-terminal tandem C2 domain, and is known to bind to Rab GTPase to participate in intracellular membrane trafficking, as well as to mediate the release of lytic granules into the cell and regulate the secretion of pancreatic and pituitary hormones.

In the present disclosure, the SYTL4 protein refers to not only the wild-type SYTL4 protein consisting of the amino acid sequence of SEQ ID No: 8, but also equivalents having the same or similar function or activity as the wild-type SYTL4 protein, although their sequences are partially different. The equivalents may include amino acid sequences having sequence homology of about 70% or more, about 75% or more, about 80% or more, about 85% or more, about 90% or more, about 92% or more, about 95% or more, about 97% or more, about 98% or more, or about 99% or more with the amino acid sequence of SEQ ID No: 8.

The composition for diagnosing radiosensitivity of breast cancer according to the present disclosure may include, as an active ingredient, an agent for measuring expression levels of mRNA of one gene, two genes, three genes, four genes, five genes, six genes, or seven genes selected from the group consisting of CD68, IFI16, TNFR2, CXCL5, LCN2, SERPING1, MATN1, and SYTL4, or proteins encoded by the genes.

In addition, the composition for predicting a prognosis of breast cancer radiotherapy according to the present disclosure may include, as an active ingredient, an agent for measuring expression levels of mRNA of one gene, two genes, three genes, four genes, five genes, six genes, or seven genes selected from the group consisting of CD68, IFI16, TNFR2, CXCL5, LCN2, SERPING1, MATN1, and SYTL4, or proteins encoded by the genes.

In a specific embodiment of the present disclosure, it was identified that the genes selected from the group consisting of CD68, IFI16, TNFR2, CXCL5, LCN2, SERPING1, MATN1, and SYTL4, or proteins encoded by the genes, have differential expression levels in radiosensitive and resistant breast cancer cells, and are closely correlated with an increased survival rate of breast cancer patients.

Accordingly, an agent for measuring expression levels of mRNA of one or two or more genes selected from the group consisting of CD68, IFI16, TNFR2, CXCL5, LCN2, SERPING1, MATN1, and SYTL4, or proteins encoded by one or two or more of the genes, may diagnose the radiosensitivity of breast cancer and predict the prognosis of breast cancer radiotherapy more effectively than mRNA and/or proteins of other genes and their combinations, and through this, the efficiency and outcome of radiotherapy for breast cancer may be improved by determining whether to perform radiotherapy and by setting an optimal radiation dose for radiotherapy.

Specifically, in breast cancer cells, the expression of the CD68, IFI16, TNFR2, CXCL5, LCN2, SERPING1, and MATN1 genes may be upregulated by radiation exposure, and the expression of the SYTL4 gene may be either upregulated or downregulated.

More specifically, the expression of the CD68, IFI16, and TNFR2 genes is upregulated in radiation-resistant breast cancer cells, and the expression of the LCN2, SERPING1, and CXCL5 genes is upregulated in radiosensitive breast cancer cells.

Furthermore, when the expression of the CD68, IFI16, TNFR2, CXCL5, LCN2, SERPING1, and MATN1 genes is upregulated in breast cancer patients, the survival rate of the breast cancer patients may be increased.

Accordingly, when the expression levels of mRNA of two or more genes selected from the group consisting of CD68, IFI16, TNFR2, CXCL5, LCN2, SERPING1, and MATN1, or proteins encoded by two or more of the genes, are higher than those of a normal control group, it may be determined that radiosensitivity is present, and when the expression levels are lower, it may be determined that radiosensitivity is low or radioresistance is present. For example, when mRNA of two to seven genes selected from the group consisting of CD68, IFI16, TNFR2, CXCL5, LCN2, SERPING1, and MATN1, and/or two to seven proteins encoded by the genes, are all highly expressed, it may be determined that radiosensitivity is high, and when the mRNA of two to seven genes and/or the two to seven proteins encoded by the genes are all lowly expressed, it may be determined that radiosensitivity is low.

Furthermore, when the expression level of mRNA of the SYTL4 gene and/or the protein encoded by the gene is significantly higher or significantly lower than that of a normal control group, it may be determined that radiosensitivity is present. The SYTL4 gene may be used as an indicator of radiation responsiveness and may be utilized to predict radiosensitivity in combination with the above genetic markers.

Meanwhile, when the expression levels of mRNA of two or more genes selected from the group consisting of CD68, IFI16, TNFR2, CXCL5, LCN2, SERPING1, MATN1, and SYTL4, or proteins encoded by the genes, are higher than those of a normal control group, the prognosis of breast cancer radiotherapy may be determined to be positive, and when the expression levels are lower, the prognosis of breast cancer radiotherapy may be determined to be negative. For example, when mRNA of two to eight genes selected from the group consisting of CD68, IFI16, TNFR2, CXCL5, LCN2, SERPING1, MATN1, and SYTL4, and/or two to eight proteins encoded by the genes, are all highly expressed, the prognosis of breast cancer radiotherapy may be determined to be positive, and when the mRNA of two to eight genes and/or the two to eight proteins encoded by the genes are all lowly expressed, the prognosis of breast cancer radiotherapy may be determined to be negative.

Meanwhile, the composition for diagnosing radiosensitivity of breast cancer and the composition for predicting a prognosis of breast cancer radiotherapy according to the present disclosure may include an agent for measuring the expression level of mRNA of the LCN2 gene or the protein encoded by the gene.

In the present disclosure, the term "radioresistance" refers to a state in which cells are not easily affected by radiation exposure and show little change in repair or proliferation when exposed to radiation. As a term opposite to the above radioresistance, "radiosensitivity" refers to a state in which cells are easily affected by radiation exposure and show changes in repair or proliferation when exposed to radiation.

In the present disclosure, the term "radiotherapy" refers to a cancer treatment that induces the death of cancer cells through a mechanism of inhibiting cell division by irradiating radiation to cause DNA damage in the cancer cells. The radiotherapy may be performed for curative, adjuvant, or palliative purposes in the treatment of solid tumors, and may be conducted alone, in combination with surgical treatment, or in combination therapy with chemotherapeutic agents or radiation sensitizers.

In the present disclosure, breast cancer may include all types such as invasive ductal carcinoma, invasive lobular carcinoma, ductal carcinoma in situ, lobular carcinoma in situ, Paget's disease of the breast, mucinous carcinoma, medullary carcinoma, inflammatory breast cancer, papillary carcinoma, tubular carcinoma, adenoid cystic carcinoma, secretory carcinoma, apocrine carcinoma, metaplastic carcinoma, estrogen receptor (ER)-positive breast cancer, HER2-positive breast cancer, and triple-negative breast cancer.

In the present disclosure, the term "diagnosis" includes determining the susceptibility of a subject to a specific disease or disorder, determining whether a subject currently has a specific disease or disorder, determining the prognosis of a subject having a specific disease or disorder, or performing therametrics (for example, monitoring the condition of a subject to provide information on therapeutic efficacy).

In the present disclosure, the term "prognosis" refers to a prospect or preliminary assessment of the medical outcome of a disease and includes a positive prognosis and a negative prognosis. The positive prognosis includes improvement or stabilization of a disease, such as remission of the disease, tumor regression, long-term survival potential, or disease-free survival rate, and the negative prognosis includes progression or fatality of the disease, such as decreased survival rate, recurrence, tumor growth, metastasis, or drug resistance.

Furthermore, in the present disclosure, the term "prediction" refers to a medical inference, for example, anticipating in advance the course of a disease (such as disease progression, improvement, recurrence, tumor growth, drug resistance, possibility of death after treatment, or survival rate) or a responsiveness to a treatment method such as chemotherapy or radiotherapy.

Specifically, in the present disclosure, the diagnosis may be a diagnosis of radiosensitivity of breast cancer, and the prognosis prediction may be a prediction of a prognosis of breast cancer radiotherapy.

In the present disclosure, the measurement of an mRNA expression level refers to a process of identifying the presence and degree of expression of the gene's mRNA in a biological sample for diagnosing the radiosensitivity of breast cancer and/or predicting a prognosis of breast cancer radiotherapy, an agent for measuring the expression level of the mRNA may include a primer, a probe, or an anti-sense nucleotide that complementarily binds to the gene or the mRNA, and the measurement of the mRNA expression level may be performed by one or more methods selected from the group consisting of RT-PCR, competitive RT-PCR, real-time RT-PCR, RNase protection assay (RPA), Northern blotting, and DNA chip analysis, but the present disclosure is not limited thereto.

In the present disclosure, the measurement of a protein expression level refers to a process of identifying the presence and degree of expression of the protein in a biological sample for diagnosing the radiosensitivity of breast cancer and/or predicting a prognosis of breast cancer radiotherapy, an agent for measuring the expression level of the protein may include a polypeptide, a compound, an antibody, or an aptamer that may specifically bind to the protein, and the measurement of the protein expression level may be performed by one or more methods selected from the group consisting of Western blotting, enzyme linked immunosorbent assay (ELISA), radioimmunoassay (RIA), radioimmunodiffusion, Ouchterlony immunodiffusion, rocket immunoelectrophoresis, immunohistochemistry, dual luciferase reporter assay, immunoprecipitation assay, complement fixation assay, fluorescence activated cell sorter (FACS), and protein chip analysis, but the present disclosure is not limited thereto.

In the present disclosure, a "sample" may refer to a sample derived from a subject who has received radiotherapy or for whom a decision on whether to receive radiotherapy is required. For example, the term "sample" refers to a sample capable of specifying expression levels of mRNA of two or more genes selected from the group consisting of CD68, IFI16, TNFR2, CXCL5, LCN2, SERPING1, MATN1, and SYTL4, or proteins encoded by two or more of the genes, and may include cells, tissues, whole blood, serum, plasma, saliva, sputum, cerebrospinal fluid, or urine of a subject, but the present disclosure is not limited thereto.

In the present disclosure, a "subject" refers to an individual to be evaluated for radiosensitivity or the possibility of progression of breast cancer, or to be predicted for a prognosis of radiotherapy. The subject is not particularly limited as long as it is an animal in which breast cancer may develop, but may specifically be a mammal, for example, a human (Homo sapiens).

In the present disclosure, the term "expression level" may be used interchangeably with "expression signature" or "expression profile," and may include one or more expression levels of mRNA of two or more genes selected from the group consisting of CD68, IFI16, TNFR2, CXCL5, LCN2, SERPING1, MATN1, and SYTL4, or proteins encoded by two or more of the genes. The expression level may include an increase or a decrease in expression, and the expression level may be measured from cancer cells isolated from a subject before and/or after radiotherapy. Meanwhile, the expression level may be identified by measuring an amount of mRNA of the gene and/or the protein in the sample.

In the present disclosure, the term "primer" refers to a short nucleic acid sequence having a free 3'-hydroxyl end, which may form base pairs with a complementary template and serves as a starting point for copying a template strand. The primer may initiate DNA synthesis in the presence of a reagent for a polymerization reaction (that is, DNA polymerase or reverse transcriptase) and four different nucleoside triphosphates, under appropriate buffer conditions and temperature. The primer of the present disclosure is a primer that may complementarily bind to the biomarker gene or mRNA of the present disclosure, and may be a sense or antisense nucleic acid having a nucleotide sequence of 7 to 50 bases, and may incorporate additional features that do not alter the essential property of the primer to serve as an initiation point for DNA synthesis. Meanwhile, the primer of the present disclosure may be chemically synthesized by using a phosphoramidite solid-support method or other well-known methods, and may be modified by using various means known in the art. Non-limiting examples of such modifications include methylation, capping, substitution with one or more analogs of natural nucleotides, and modifications between nucleotides, for example, modification to uncharged linkages (e.g., methyl phosphonate, phosphotriester, phosphoramidate, carbamate, etc.) or charged linkages (e.g., phosphorothioate, phosphorodithioate, etc.). The nucleic acid may contain one or more additional covalently bound residues, such as a protein (e.g., nuclease, toxin, antibody, signal peptide, or poly-L-lysine), an intercalator (e.g., acridine or psoralen), a chelating agent (e.g., metal, radioactive metal, iron, or oxidized metal), and an alkylating agent. In addition, the primer of the present disclosure may be modified by using a label capable of providing a detectable signal directly or indirectly, such as a radioactive isotope, a fluorescent molecule, or biotin.

In the present disclosure, the term "probe" refers to a nucleic acid that may complementarily bind to mRNA and is produced through enzymatic or chemical purification or synthesis processes, having a length of several to hundreds of bases. The probe of the present disclosure is a probe capable of complementarily binding to the biomarker gene or mRNA of the present disclosure, and may be labeled with a radioactive isotope or an enzyme to identify the presence or absence of mRNA, and may be designed and modified by known methods.

In the present disclosure, the term "antisense nucleotide" refers to DNA, RNA, or a derivative thereof containing a nucleic acid sequence complementary to the sequence of a specific mRNA, and binds to the complementary sequence within the mRNA and inhibits the translation of the mRNA into a protein. The sequence of the antisense nucleotide refers to a DNA or RNA sequence that is complementary to the mRNA of the biomarker gene of the present disclosure, selected from the group consisting of CD68, IFI16, TNFR2, CXCL5, LCN2, SERPING1, MATN1, and SYTL4, and that may bind to the mRNA. This may inhibit an essential activity of the mRNA in translation, translocation into the cytoplasm, maturation, or any other overall biological function. The antisense nucleotide may be synthesized in vitro by conventional methods and administered in vivo, or may be produced in vivo, and the antisense nucleotide usable in the present disclosure may be prepared according to methods known in the art, with reference to the nucleotide sequence and/or amino acid sequence of the biomarker gene and/or protein of the present disclosure.

In the present disclosure, the term "antibody" refers to a globulin-type protein that circulates in blood or lymph within the immune system of a living organism and reacts to an invading external substance, that is, an antigen, and means a protein that specifically binds to the antigen. For the purposes of the present disclosure, the term "antibody" refers to an antibody that specifically binds to the biomarker protein encoded by a gene selected from the group consisting of CD68, IFI16, TNFR2, CXCL5, LCN2, SERPING1, MATN1, and SYTL4, includes polyclonal antibodies, monoclonal antibodies, and recombinant antibodies, and includes not only the complete form having two full-length light chains and two full-length heavy chains, but also functional fragments of antibody molecules. The functional fragment of an antibody molecule refers to a fragment that retains at least an antigen-binding function, and examples thereof include Fab, F(ab'), F(ab')2, and Fv. An antibody that specifically binds to the biomarker protein of the present disclosure may be produced by methods known to those skilled in the art, for example, by injecting the immunogenic biomarker protein into an external host. The external host may include mammals such as mice, rats, sheep, and rabbits, and the immunogen may be injected by intramuscular, intraperitoneal, or subcutaneous injection, and is generally administered together with an adjuvant to enhance antigenicity. Thereafter, blood may be periodically collected from the external host, and serum showing specificity to the antigen may be obtained, from which the antibody may be isolated.

In the present disclosure, the term "aptamer" is a single-stranded oligonucleotide, has a length of about 20 to 60 nucleotides, and refers to a nucleic acid molecule having a binding activity to a specific target molecule. The aptamer has various three-dimensional structures depending on its sequence, has high affinity for specific substances like an antigen-antibody reaction, and binds to a specific target molecule so that it may inhibit the activity of the specific target molecule. The aptamer may be RNA, DNA, a modified nucleic acid, or a mixture thereof, and may also be in a linear or circular form. In the present disclosure, the aptamer may specifically bind to the biomarker protein encoded by a gene selected from the group consisting of CD68, IFI16, TNFR2, CXCL5, LCN2, SERPING1, MATN1, and SYTL4, and may be prepared by a method known to those skilled in the art based on the nucleotide sequence of the biomarker gene of the present disclosure.

### 2. Kit for Diagnosing Radiosensitivity of Breast Cancer and Kit for Predicting Prognosis of Breast Cancer Radiotherapy

Another aspect of the present disclosure provides a kit for diagnosing radiosensitivity of breast cancer.

Furthermore, another aspect of the present disclosure provides a kit for predicting a prognosis of breast cancer radiotherapy.

The kit for diagnosing radiosensitivity of breast cancer according to the present disclosure includes, as an active ingredient, an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

In addition, the composition for predicting a prognosis of breast cancer radiotherapy according to the present disclosure includes, as an active ingredient, an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

As described above in connection with the composition for diagnosing radiosensitivity of breast cancer or for predicting a prognosis of breast cancer radiotherapy, a repeated description thereof will be omitted.

In the present disclosure, the term "kit" refers to a tool that may diagnose radiosensitivity of breast cancer or predict a prognosis of breast cancer radiotherapy by identifying expression levels of mRNA of two or more genes selected from the group consisting of CD68, IFI16, TNFR2, CXCL5, LCN2, SERPING1, MATN1, and SYTL4, or proteins encoded by two or more of the genes. The kit of the present disclosure may include an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes, and specifically, primers, probes, and antisense nucleotides that specifically bind to the mRNA of the genes, or polypeptides, compounds, antibodies, and aptamers that specifically bind to the proteins, and may further include one or more other compositions, solutions, or devices suitable for the analytical method.

Furthermore, the kit may additionally include a user manual that describes optimal reaction conditions. The manual may include an instruction booklet in the form of a pamphlet or leaflet, a label attached to the kit, or a description on the surface of a package containing the kit, and may also include information disclosed or provided through an electronic medium such as the Internet.

In the present disclosure, the kit may be an RT-PCR kit, a microarray chip kit, a DNA kit, an ELISA kit, a protein chip kit, or a rapid kit.

### 3. Method for Providing Information for Diagnosing Radiosensitivity of Breast Cancer and Method for Providing Information for Predicting Prognosis of Breast Cancer Radiotherapy

Another aspect of the present disclosure provides a method for providing information for diagnosing radiosensitivity of breast cancer.

Furthermore, another aspect of the present disclosure provides a method for providing information for predicting a prognosis of breast cancer radiotherapy.

The method for providing information for diagnosing radiosensitivity of breast cancer according to the present disclosure includes (a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes, and (b) comparing the expression levels of the mRNA or proteins with expression levels of mRNA or proteins of a normal control group.

The method for providing information for predicting a prognosis of breast cancer radiotherapy according to the present disclosure includes, (a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes, and (b) comparing the expression levels of the mRNA or proteins with expression levels of mRNA or proteins of a normal control group.

As described above in connection with the composition for diagnosing radiosensitivity of breast cancer or for predicting a prognosis of breast cancer radiotherapy, a repeated description thereof will be omitted.

In the method for providing information for diagnosing radiosensitivity of breast cancer according to the present disclosure, (a) may include measuring an expression level of mRNA of the LCN2 gene or a protein encoded by the gene.

Furthermore, in the method for providing information for predicting a prognosis of breast cancer radiotherapy according to the present disclosure, (a) may include measuring an expression level of mRNA of the LCN2 gene or a protein encoded by the gene.

In the method for providing information for diagnosing radiosensitivity of breast cancer according to the present disclosure, in (b), when expression levels of mRNA of two or more genes selected from the group consisting of CD68, IFI16, TNFR2, CXCL5, LCN2, SERPING1, and MATN1, or proteins encoded by two or more of the genes, are compared with expression levels of mRNA or proteins of a normal control group, and are found to be higher than those of the normal control group, it may be determined that the breast cancer has radiosensitivity. The expression that the expression level is high may include that an expression level of a subject is at a similar level to, or increased by 1%, 2%, 3%, 4%, 5%, 10%, 20%, 30%, 40%, 50%, 60%, 70%, 80%, 90%, 100%, 200%, 300%, 400%, 500%, 600%, 700%, 800%, 900%, or 1000% or more compared with an expression level of a normal control group.

Furthermore, in the method for providing information for diagnosing radiosensitivity of breast cancer according to the present disclosure, in (b), when an expression level of mRNA of the SYTL4 gene, or a protein encoded by the gene, is compared with an expression level of mRNA or a protein of a normal control group, and is found to be higher or lower than that of the normal control group, it may be determined that the breast cancer has radiosensitivity. The expression that the expression level is high may include that an expression level of a subject is at a similar level to, or increased by 1%, 2%, 3%, 4%, 5%, 10%, 20%, 30%, 40%, 50%, 60%, 70%, 80%, 90%, 100%, 200%, 300%, 400%, 500%, 600%, 700%, 800%, 900%, or 1000% or more compared with an expression level of a normal control group, and the expression that the expression level is low may include that an expression level of a subject is at a similar level to, or decreased by 1%, 2%, 3%, 4%, 5%, 10%, 20%, 30%, 40%, 50%, 60%, 70%, 80%, 90%, or 99% or more compared with an expression level of a normal control group.

Meanwhile, in the method for providing information for predicting a prognosis of breast cancer radiotherapy according to the present disclosure, in the step (b), when expression levels of mRNA of two or more genes selected from the group consisting of CD68, IFI16, TNFR2, CXCL5, LCN2, SERPING1, MATN1, and SYTL4, or proteins encoded by two or more of the genes, are compared with expression levels of mRNA or proteins of a normal control group, and are found to be higher than those of the normal control group, it may be determined that the prognosis of the breast cancer radiotherapy is positive. The expression that the expression level is high may include that an expression level of a subject is at a similar level to, or increased by 1%, 2%, 3%, 4%, 5%, 10%, 20%, 30%, 40%, 50%, 60%, 70%, 80%, 90%, 100%, 200%, 300%, 400%, 500%, 600%, 700%, 800%, 900%, or 1000% or more compared with an expression level of a normal control group.

Meanwhile, in the method for providing information for diagnosing radiosensitivity of breast cancer according to the present disclosure, the method may further include (c-1) determining that the breast cancer has radiosensitivity when expression levels of mRNA of two or more genes selected from the group consisting of CD68, IFI16, TNFR2, CXCL5, LCN2, SERPING1, and MATN1, or proteins encoded by two or more of the genes, are higher than those of a normal control group, and/or (c-2) determining that the breast cancer has radiosensitivity when an expression level of mRNA of the SYTL4 gene, or a protein encoded by the gene, is higher or lower than that of a normal control group.

Furthermore, in the method for providing information for predicting a prognosis of breast cancer radiotherapy according to the present disclosure, the method may further include (c) determining that the prognosis of the breast cancer radiotherapy is positive when a measured expression level of mRNA or a protein is higher than that of a normal control group.

In a specific embodiment of the present disclosure, genes or combinations thereof having a P-value of 0.05 or less were identified by analyzing overall survival (OS) using TCGA data, and HR (hazard ratio) values according to each gene or combination were identified. The HR represents the risk, and indicates that the higher the HR value in the high gene expression group, the poorer the survival rate of patients, and the lower the HR value, the higher the survival rate of patients. When the HR is 0.8 or less, 0.7 or less, 0.6 or less, 0.5 or less, or 0.4 or less, the prognosis of the breast cancer radiotherapy of a subject may be determined to be positive.

Another aspect of the present disclosure provides a method for diagnosing radiosensitivity of breast cancer, including (a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes; and (b) comparing the expression levels of the mRNA or proteins with expression levels of mRNA or proteins of a normal control group.

Another aspect of the present disclosure provides a method for predicting a prognosis of breast cancer radiotherapy, including (a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes; and (b) comparing the expression levels of the mRNA or proteins with expression levels of mRNA or proteins of a normal control group.

Another aspect of the present disclosure provides a use of an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes, for diagnosing radiosensitivity of breast cancer.

Another aspect of the present disclosure provides a use of an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes, for predicting a prognosis of breast cancer radiotherapy.

### [Advantageous Effects]

In the present disclosure, mRNA of the CD68, IFI16, TNFR2, CXCL5, LCN2, SERPING1, MATN1, and SYTL4 genes, or proteins encoded by the genes, have differential expression levels in radioresistant and radiosensitive breast cancer cells, and have a close correlation with an increased survival rate of breast cancer patients. Accordingly, an agent for measuring an expression level of mRNA of a gene selected from the group consisting of CD68, IFI16, TNFR2, CXCL5, LCN2, SERPING1, MATN1, and SYTL4, or a protein encoded by the gene, according to the present disclosure, may effectively diagnose radiosensitivity of breast cancer or predict a prognosis of breast cancer radiotherapy, and, through this, may improve efficiency and outcomes of radiotherapy for breast cancer by determining whether to perform radiotherapy and an optimal radiation dose for the radiotherapy.

However, the effects of the present disclosure are not limited to those mentioned above, and other effects not mentioned will be clearly understood by those skilled in the art from the following description.

### [Description of Drawings]

FIG. 1a shows results of identifying cell death according to radiation exposure in human breast cancer cell lines SK-BR3, T47D, MDA-MB-231, and BT-549 by fluorescence-activated cell sorting (FACS).
FIG. 1b shows results of measuring colony-forming ability according to radiation exposure in human breast cancer cell lines SK-BR3, T47D, MDA-MB-231, and BT-549.
FIG. 2 shows results of analyzing genes whose expression increased or decreased after exposure to 8 Gy of radiation in human breast cancer cell lines SK-BR3, T47D, MDA-MB-231, and BT-549.
FIG. 3 shows results of analyzing changes in the expression of proteins of radiosensitivity-related genes in human breast cancer cell lines SK-BR3, T47D, MDA-MB-231, and BT-549.
FIG. 4 shows results of analyzing expression levels of radiosensitivity-related genes by using RNA-seq data (GSE65505) from 62 breast cancer patients who received radiotherapy through a DESeq2 program.
FIG. 5a shows Kaplan-Meier survival curves for survival rates of breast cancer patients according to expression of LCN2, SYTL4, or CXCL5.
FIG. 5b shows Kaplan-Meier survival curves for survival rates of breast cancer patients according to expression of LCN2 and CXCL5, LCN2 and SYTL4, or SYTL4 and CXCL5.
FIG. 5c shows Kaplan-Meier survival curves for survival rates of breast cancer patients according to expression of SYTL4, CXCL5, and TNFR2, or expression of SYTL4, CXCL5, TNFR2, and MATN1.
FIG. 6a shows results of identifying cancer cell death according to presence or absence of radiation exposure after knocking down expression of radiosensitivity-related genes with siRNA in a human breast cancer cell line MDA-MB-231.
FIG. 6b shows results of identifying cancer cell death according to presence or absence of radiation exposure in a human breast cancer cell line MDA-MB-231 after single knockdown of CXCL5, double knockdown of LCN2 or SYTL4 with CXCL5, or triple knockdown of CXCL5, SYTL4, and TNFR2.

### [Mode for Invention]

Hereinafter, the present disclosure will be described in detail with reference to examples.

However, the following examples are provided to specifically illustrate the present disclosure, and the scope of the present disclosure is not limited by the following examples.

### Example 1. Screening of Radiosensitive or Radioresistant Breast Cancer Cell Lines

Among human breast cancer cell lines, four types of HER2-positive SK-BR3 (ER⁻/PR⁻/HER2⁺) cell lines, hormone receptor-positive luminal A-type T47D (ER⁺/PR⁺/HER2⁻) cell lines, and triple-negative (ER⁻/PR⁻/HER2⁻) breast cancer cell lines MDA-MB-231 and BT-549 were purchased from the American Type Culture Collection (ATCC, USA) and used, and were maintained in RPMI-1640 medium supplemented with 10% fetal bovine serum (FBS) and 1% penicillin-streptomycin (10,000 U/ml) under conditions of 5% CO₂ and 37 °C. Thereafter, to measure the radioresponsiveness of the breast cancer cell lines, the cells were exposed to 8 Gy of radiation, and after 24 hours, the degree of cell death was analyzed by fluorescence-activated cell sorting (FACS) through Annexin V/PI staining.

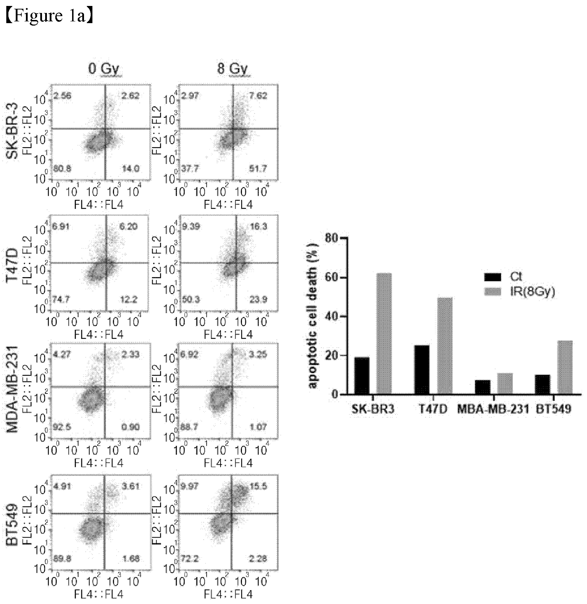

As a result, it was identified that the SK-BR3 and T47D cell lines showed a significant increase in cell death upon radiation exposure and exhibited radiosensitivity, and the MDA-MB-231 and BT-549 cell lines showed a lesser increase in cell death upon radiation exposure and exhibited radioresistance (FIG. 1a).

Next, the breast cancer cell lines were seeded at an appropriate cell density in 60-mm dishes, and after 24 hours, the cells were exposed to radiation at various doses and then cultured for 10-14 days under conditions of 5% CO₂ and 37 °C. Thereafter, the formed cell colonies were washed with phosphate-buffered saline (PBS) and stained with a mixture of 100% methanol containing 1% methylene blue. The stained cell colonies (colonies containing 50 or more cells) were air-dried at room temperature and visually counted for each dish.

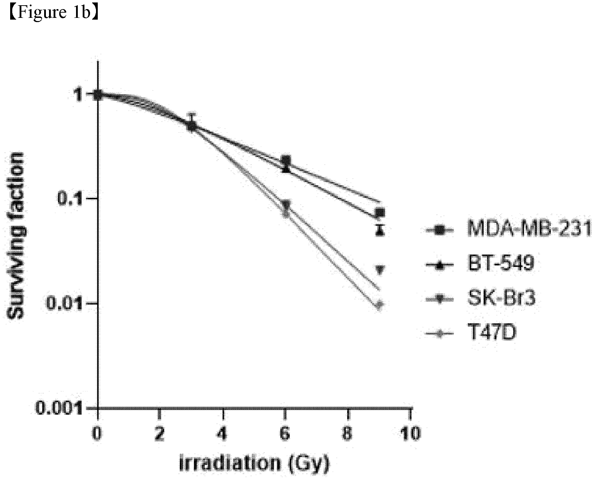

As a result, it was identified that the SK-BR3 and T47D cell lines showed a significant decrease in survival rate upon radiation exposure and exhibited radiosensitivity, and the MDA-MB-231 and BT-549 cell lines did not show a substantial decrease in survival rate even after radiation exposure and exhibited radioresistance (FIG. 1b).

### Example 2. Identification of Genes Associated with Radiosensitivity in Breast Cancer

To identify genes associated with radiosensitivity in breast cancer, genes were screened based on transcriptome and secreted protein analysis results according to the presence or absence of radiation exposure, and the radioresponsiveness of each gene was measured by real-time reverse transcription polymerase chain reaction (real-time RT-PCR). The primer information used in the present example is as in Table 1 below.

**[Table 1]**

| **Target Sequence** | **Official Gene Name** | **Product Length** | **Dir.** | **Sequence (5'->3')** |
|---|---|---|---|---|
| NM_001040059 .2 | CD68 | 136 | Forward | CGA GCA TCA TTC TTT CAC CAG CT (Sequence ID No. 9) |
| | | | Reverse | ATG AGA GGC AGC AAG ATG GAC C (Sequence ID No. 10) |
| NM_001364867 .2 | IFI16 | 134 | Forward | GAT GCC TCC ATC AAC ACC AAG C (Sequence ID No. 11) |
| | | | Reverse | CTG TTG CGT TCA GCA CCA TCA C (Sequence ID No. 12) |
| NM_001066.3 | TNFRS F1B | 102 | Forward | CGT TCT CCA ACA CGA CTT CAT CC (Sequence ID No. 13) |
| | | | Reverse | ACG TGC AGA CTG CAT CCA TGC T (Sequence ID No. 14) |
| NM_005564.5 | LCN2 | 112 | Forward | GTG AGC ACC AAC TAC AAC CAG C (Sequence ID No. 15) |
| | | | Reverse | GTT CCG AAG TCA GCT CCT TGG T (Sequence ID No. 16) |
| NM_000062.3 | SERPIN G1 | 157 | Forward | GCA TCA AAG TGA CGA CCA GCC A (Sequence ID No. 17) |
| | | | Reverse | GTC TCT GTC AGT TCC AGC ACT G (Sequence ID No. 18) |
| NM_002994.5 | CXCL5 | 111 | Forward | CAG ACC ACG CAA GGA GTT CAT C (Sequence ID No. 19) |
| | | | Reverse | TTC CTT CCC GTT CTT CAG GGA G (Sequence ID No. 20) |
| NM_002379.3 | MATN1 | 92 | Forward | GGC TTC ACT CTG AAC AGC GAC G (Sequence ID No. 21) |
| | | | Reverse | CCG TCA ATG AGG AAG ACC AGG T (Sequence ID No. 22) |
| NM_001129896 .3 | SYTL4 | 94 | Forward | GGA GAC AGA AGC AAA TCC GTC C (Sequence ID No. 23) |
| | | | Reverse | GGC GAT GTA ACT TCA CTA GGT GG (Sequence ID No. 24) |
| NM_022551.3 | RPS18 | 107 | Forward | CAC GCC AGT ACA AGA TCC CA (Sequence ID No. 25) |
| | | | Reverse | TTC ACG GAG CTT GTT GTC CA (Sequence ID No. 26) |

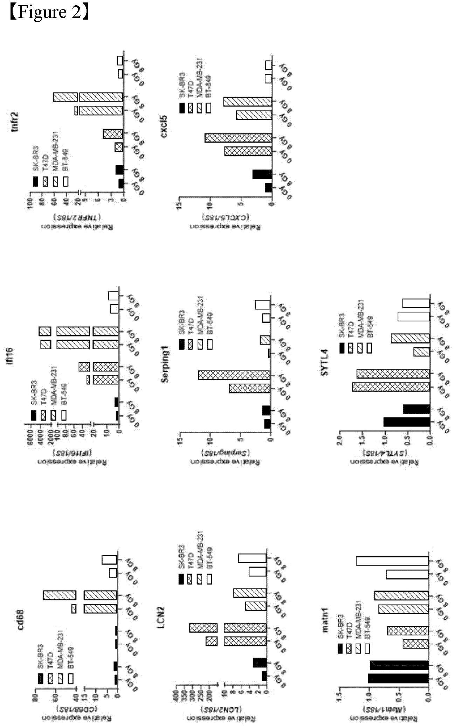

Subsequently, four breast cancer cell lines were exposed to 0 or 8 Gy of radiation, and the expression of each gene was measured 24 hours after the exposure. As a result, the expression of CD68, IFI16, TNFR2, CXCL5, LCN2, SERPING1, and MATN1 increased upon radiation exposure, whereas SYTL4 showed a significant increase in expression in MDA-MB-231 but decreased in other cell lines, indicating that radiation exposure led to either an increase or a decrease in gene expression depending on the cell line (FIG. 2). Specifically, CD68, IFI16, and TNFR2 showed high expression in the radioresistant cell line MDA-MB-231, while LCN2, SERPING1, and CXCL5 exhibited increased expression in all four cell lines upon radiation exposure, with a particularly significant increase observed in the T47D cell line.

### Example 3. Identification of Protein Expression Changes in Radioresponsiveness-Related Factors

To identify the protein expression patterns of the radiosensitivity-related genes derived from Example 2 according to radiation exposure, SK-BR3, T47D, MDA-MB-231, and BT-549 cell lines were exposed to 8 Gy of radiation, and after 24 hours, proteins were extracted and analyzed by Western blotting assay. The antibody information used in the present example is as in Table 2 below.

**[Table 2]**

| Primary Antibody | Manufacturer | Cat.No | Host | Size (kD) |
|---|---|---|---|---|
| CD68 | Santa Cruz | sc-20060 | Mouse | 75-100 |
| IFI16 | Santa Cruz | sc-8023 | Mouse | 85-95 |
| TNFR2 | Invitrogen | MA5-32618 | Rabbit | 60 |
| LCN2 | Invitrogen | PA5-79589 | Rabbit | 22 |
| SERPING1 | Invitrogen | PA5-31541 | Rabbit | 100 |
| CXCL5 | Invitrogen | 700656 | Rabbit | 8 |
| MATN1 | Invitrogen | MA5-15704 | Mouse | 43 |
| SYTL4 | Invitrogen | PA5-35992 | Rabbit | 80-90 |
| ACTB | Sigma | A5316 | Mouse | 42 |

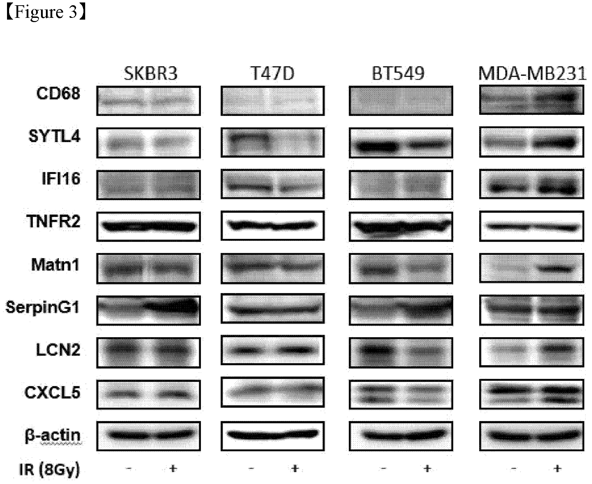

As a result, it was shown that the expression levels of CD68, SYTL4, IFI16, MATN1, LCN2, and CXCL5 proteins were significantly increased in the MDA-MB-231 cell line, and a significant increase in SERPING1 protein expression was observed in the SK-BR3 and BT-549 cell lines (FIG. 3). Meanwhile, TNFR2 protein showed high expression across all breast cancer cell lines, and SYTL4 protein, similar to its gene expression pattern, exhibited increased expression in the MDA-MB-231 cell line but showed a significant decrease in expression upon radiation exposure in the other cell lines.

### Example 4. Analysis of Risk and Survival Curves According to Expression of Radiosensitivity Gene Panel in Breast Cancer Patients

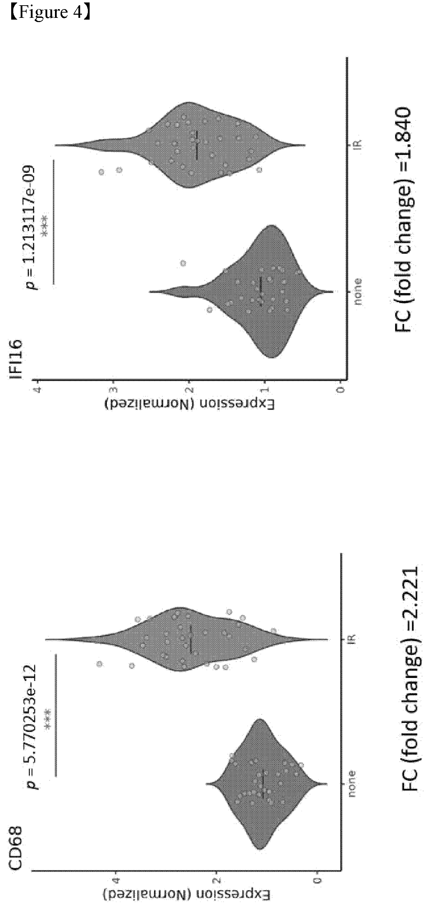

To identify the expression patterns of the radiosensitivity-related genes derived from Example 2 in actual breast cancer patients, RNA-seq data (GSE65505) from 62 breast cancer patients who received radiotherapy were downloaded and analyzed by using the DESeq2 program. As a result, it was shown that CD68 expression was significantly increased by 2.221-fold compared with the normal control group, and IFI16 expression was significantly increased by 1.84-fold compared with the normal control group (FIG. 4).

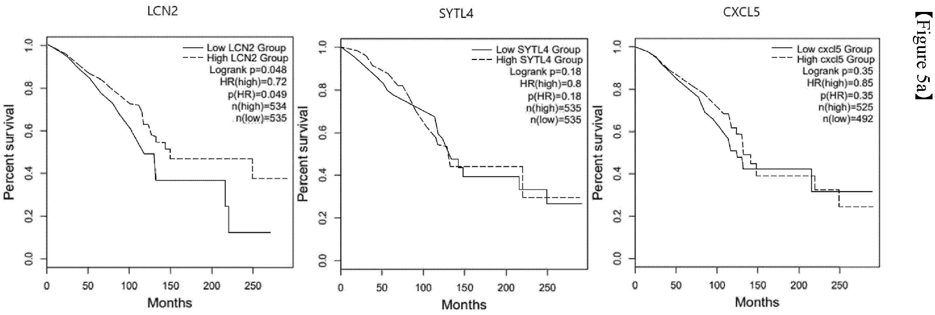

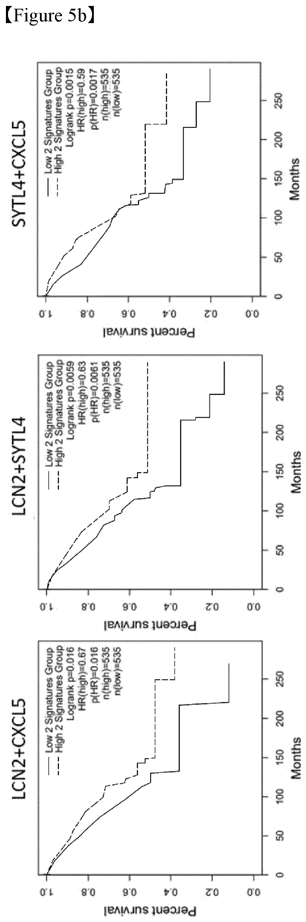

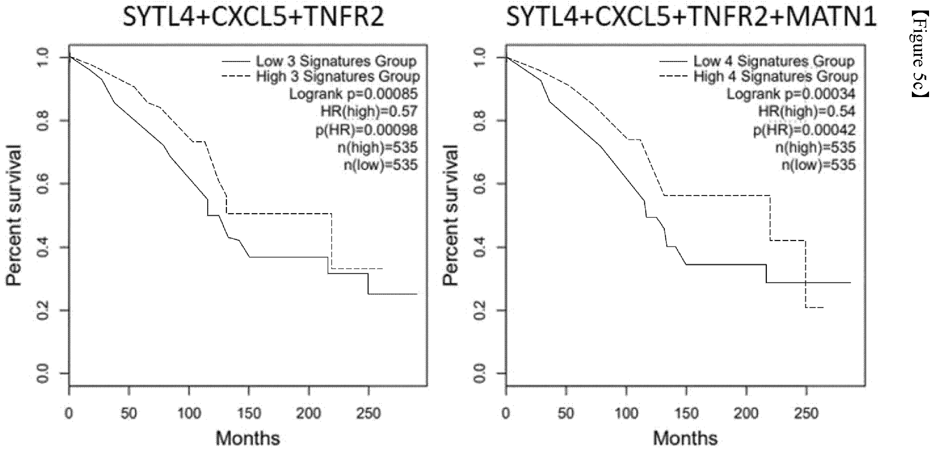

Thereafter, to analyze the effects of the expression of the radiosensitivity-related genes derived from Example 2 on the survival rate of breast cancer patients, overall survival (OS) was analyzed by utilizing the Cancer Genome Atlas (TCGA) database that is a published gene database material through GEPIA2, and genes or combinations showing a P-value of 0.05 or less were identified, and the results are shown in Table 3 below. In Table 3 below, hazard ratio (HR) represents the risk, and indicates that the higher the HR value in the high gene expression group, the poorer the survival rate of patients, and the lower the HR value, the higher the survival rate of patients. In addition, the survival rates of breast cancer patients according to the expression of the genes or combinations thereof were analyzed and visualized through Kaplan-Meier survival curves (FIGS. 5a to 5c).

**[Table 3]**

| **Combination** | | | | | | | | **HR** | **P-value** |
|---|---|---|---|---|---|---|---|---|---|
| LCN2 | | | | | | | | 0.72 | 0.049 |
| LCN2 | MATN1 | | | | | | | 0.72 | 0.05 |
| SYTL4 | VTN | CXCL5 | MATN1 | | | | | 0.72 | 0.048 |
| SYTL4 | VTN | CXCL5 | MATN1 | LCN2 | | | | 0.72 | 0.046 |
| TNFR2 | CXCL5 | IFI16 | | | | | | 0.71 | 0.038 |
| TNFR2 | CXCL5 | | | | | | | 0.71 | 0.037 |
| SYLT4 | VTN | CXCL5 | TNFR2 | | | | | 0.7 | 0.036 |
| SYTL4 | VTN | CXCL5 | CD68 | | | | | 0.7 | 0.036 |
| SYLT4 | VTN | CXCL5 | LCN2 | | | | | 0.7 | 0.032 |
| SYTL4 | CXCL5 | CD68 | | | | | | 0.7 | 0.032 |
| SYTL4 | TNFR2 | CD68 | | | | | | 0.69 | 0.03 |
| TNFR2 | Serping 1 | | | | | | | 0.69 | 0.029 |
| SYTL4 | LCN2 | TNFR2 | | | | | | 0.69 | 0.026 |
| LCN2 | CXCL5 | MATN1 | | | | | | 0.69 | 0.023 |
| SYTL4 | LCN2 | CXCL5 | MATN1 | CD68 | | | | 0.68 | 0.022 |
| SYTL4 | VTN | LCN2 | CD68 | | | | | 0.68 | 0.019 |
| SYTL4 | VTN | CXCL5 | MATN1 | CD68 | | | | 0.67 | 0.019 |
| TNFR2 | CXCL5 | IFI16 | Serping 1 | | | | | 0.67 | 0.018 |
| SYTL4 | LCN2 | CXCL5 | CD68 | | | | | 0.67 | 0.016 |
| LCN2 | CXCL5 | | | | | | | 0.67 | 0.016 |
| TNFR2 | SYLT4 | VTN | LCN2 | MATN1 | | | | 0.67 | 0.015 |
| TNFR2 | SYTL4 | MATN1 | CD68 | | | | | 0.66 | 0.018 |
| SYTL4 | VTN | LCN2 | CXCL5 | CD68 | | | | 0.66 | 0.014 |
| SYTL4 | CXCL5 | MATN1 | CD68 | | | | | 0.66 | 0.013 |
| TNFR2 | CXCL5 | Serping 1 | | | | | | 0.65 | 0.011 |
| SYLT4 | LCN2 | VTN | CD68 | TNFR2 | | | | 0.65 | 0.011 |
| SYTL4 | LCN2 | VTN | CXCL5 | MATN1 | CD68 | | | 0.65 | 0.01 |
| SYTL4 | LCN2 | MATN1 | | | | | | 0.65 | 0.0087 |
| SYTL4 | LCN2 | MATN1 | TNFR2 | | | | | 0.64 | 0.0083 |
| SYTL4 | LCN2 | CXCL5 | | | | | | 0.64 | 0.0072 |
| SYLT4 | LCN2 | VTN | TNFR2 | | | | | 0.64 | 0.0069 |
| SYTL4 | LCN2 | CXCL5 | MATN1 | | | | | 0.64 | 0.0065 |
| SYTL4 | LCN2 | CD68 | | | | | | 0.63 | 0.0062 |
| SYTL4 | LCN2 | | | | | | | 0.63 | 0.0061 |
| SYTL4 | TNFR2 | LCN2 | CXCL5 | | | | | 0.63 | 0.0052 |
| SYTL4 | TNFR2 | LCN2 | VTN | CXCL5 | CD68 | | | 0.62 | 0.0058 |
| SYTL4 | TNFR2 | LCN2 | CXCL5 | MATN1 | | | | 0.62 | 0.0038 |
| SYTL4 | TNFR2 | LCN2 | VTN | CXCL5 | MATN1 | CD68 | | 0.61 | 0.0045 |
| SYTL4 | TNFR2 | LCN2 | VTN | CXCL5 | MATN1 | | | 0.61 | 0.0038 |
| SYTL4 | TNFR2 | LCN2 | CD68 | | | | | 0.61 | 0.0037 |
| SYLT4 | TNFR2 | | | | | | | 0.6 | 0.0032 |
| SYTL4 | TNFR2 | LCN2 | CXCL5 | MATN1 | CD68 | | | 0.6 | 0.0026 |
| SYTL4 | TNFR2 | CXCL5 | CD68 | | | | | 0.6 | 0.0025 |
| SYTL4 | TNFR2 | CXCL5 | MATN1 | CD68 | | | | 0.6 | 0.0027 |
| SYTL4 | TNFR2 | LCN2 | CXCL5 | CD68 | | | | 0.59 | 0.002 |
| SYTL4 | TNFR2 | VTN | LCN2 | CXCL5 | | | | 0.59 | 0.0018 |
| SYTL4 | CXCL5 | | | | | | | 0.59 | 0.0017 |
| SYTL4 | TNFR2 | CXCL5 | | | | | | 0.57 | 0.00098 |
| SYTL4 | TNFR2 | MATN1 | | | | | | 0.56 | 0.00077 |
| SYTL4 | CXCL5 | MATN1 | | | | | | 0.55 | 0.00034 |
| SYTL4 | TNFR2 | CXCL5 | MATN1 | | | | | 0.54 | 0.00042 |

As a result of the analysis, it was shown that a higher expression of LCN2 reduced the risk of breast cancer patients to 0.72, thereby increasing the survival rate. Meanwhile, it was shown that simultaneous increases in the expression of the LCN2 and CXCL5 genes, the LCN2 and SYTL4 genes, or the SYTL4 and CXCL5 genes reduced the risk to 0.67, 0.63, and 0.59, respectively. It was shown that when the three-gene combination of SYTL4/CXCL5/TNFR2 or the four-gene combination of SYTL4/CXCL5/TNFR2/MATN1 was present, the risk decreased to 0.57 and 0.54, respectively, thereby increasing the survival rate of breast cancer patients.

### Example 5. Analysis of Cell Survival Following Knockdown of Radiation-Responsive Genes in Human Breast Cancer Cells

It was identified whether a change in the survival rate of breast cancer cells occurred upon radiation exposure when the expression of the radiosensitivity genes derived from Example 2 was knocked down. To this end, after knocking down the expression of the radiosensitivity genes by using siRNA, radiation exposure was applied to the breast cancer cell line MDA-MB-231, and changes in their survival rate were measured.

Specifically, each siRNA was transfected into the breast cancer cell line using Lipofectamine^{™} RNAiMAX transfection reagent (Thermo Fisher Scientific, USA) in a 96-well plate, followed by radiation exposure at a dose of 8 Gy. Thereafter, after 24 hours, Annexin V/PI (BD Pharmingen) reagent was treated, cell apoptosis was analyzed by FACS, and the results were plotted as graphs. The siRNA information used in the present example is as in Table 4 below.

**[Table 4]**

| **Official Gene Name** | **NCBI Gene ID** | **Direction** | **Sequence (5'->3')** |
|---|---|---|---|
| Scramble RNA | | Sense | CCUCGUGCCGUUCCAUCAGGUAG (SEQ ID No: 27) |
| | | Anti-sense | CUACCUGAUGGAACGGCACGAGG (SEQ ID No: 28) |
| LCN2 | 3934 | Sense | UGUCCCAAUCGACCAGUGUAU (SEQ ID No: 29) |
| | | Anti-sense | AUACACUGGUCGAUUGGGACA (SEQ ID No: 30) |
| TNFRSF1B | 8784 | Sense | GCCUUGGGUCUACUAAUAAUA (SEQ ID No: 31) |
| | | Anti-sense | UAUUAUUAGUAGACCCAAGGC (SEQ ID No: 32) |
| MATN1 | 4146 | Sense | CGGCUUUAAGAUGUUUGCUGU (SEQ ID No: 33) |
| | | Anti-sense | ACAGCAAACAUCUUAAAGCCG (SEQ ID No: 34) |
| CXCL5 | 6374 | Sense | UGAAUUGUAGGUGACUAUUAU (SEQ ID No: 35) |
| | | Anti-sense | AUAAUAGUCACCUACAAUUCA (SEQ ID No: 36) |
| SYTL4 | 94121 | Sense | GAUGAGGGUGAGAUGAUAUUU (SEQ ID No: 37) |
| | | Anti-sense | AAAUAUCAUCUCACCCUCAUC (SEQ ID No: 38) |

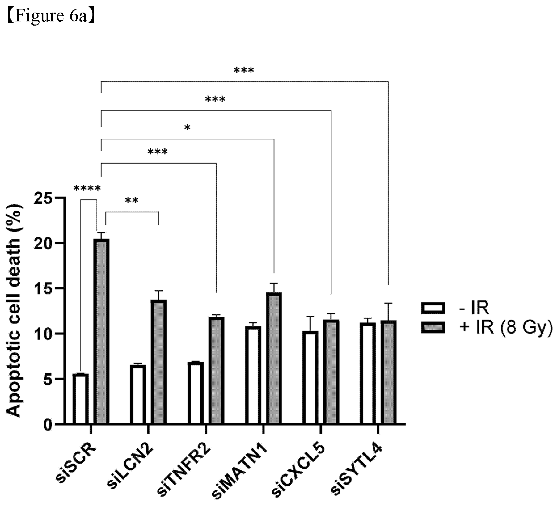

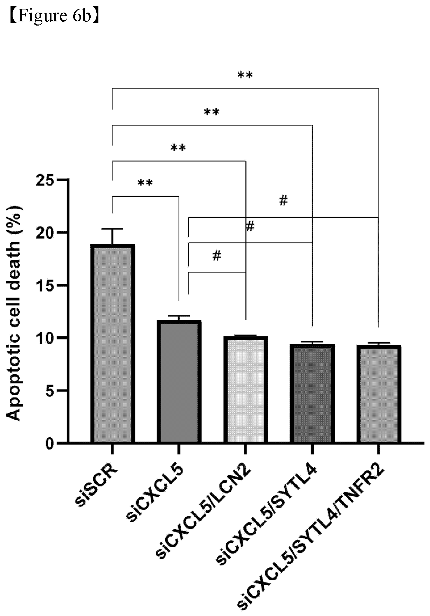

As a result, when each gene was knocked down compared with the control group, in which a scramble RNA (siSCR) having a non-specific sequence was treated as a control group, significant decreases in the cell survival rate were observed in the MDA-MB-231 cell line by single knockdown of MATN1, CXCL5, and SYTL4, and LCN2 and TNFR2 did not show significant changes by single gene knockdown (FIG. 6a). When cell death was observed after combining radiation exposure under the same conditions, a remarkable decrease in cell death was found upon knockdown of CXCL5, SYTL4, and TNFR2 compared with the control group, thereby identifying these genes as radiosensitivity genes of breast cancer. Furthermore, knockdown of the LCN2 and MATN1 genes also showed a reduction effect on cell death induced by radiation. Furthermore, to identify the radiosensitivity effect according to co-knockdown of the genes, radiation was exposed after single knockdown of CXCL5, double knockdown of CXCL5/LCN2 or CXCL5/SYTL4, or triple knockdown of CXCL5/SYTL4/TNFR2, and after 24 hours, cell death was measured through FACS analysis. As a result, similar to the results of Example 4, a more significant inhibitory effect on cell death was shown in the groups subjected to double knockdown of CXCL5/LCN2 or CXCL5/SYTL4, or triple knockdown of CXCL5/SYTL4/TNFR2, than in the group with single knockdown of CXCL5 (FIG. 6b).

Through the above experiment, it was identified that the sensitivity of breast cancer cells to radiotherapy and the prognosis of radiotherapy could be effectively predicted by measuring the expression of CD68, IFI16, TNFR2, CXCL5, LCN2, SERPING1, MATN1, and SYTL4. Accordingly, it is expected that breast cancer may be treated more effectively when radiotherapy is combined while identifying the activity of the above radiosensitivity gene panel.

The representative examples of the present disclosure have been described above by way of example, but the scope of the present disclosure is not limited to the specific embodiments described above, and those skilled in the art will be able to make appropriate modifications within the scope set forth in the claims of the present disclosure.

## Claims

1. A composition for diagnosing radiosensitivity of breast cancer, the composition comprising: an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

2. The composition of claim 1, wherein the composition comprises an agent for measuring an expression level of mRNA of an LCN2 gene, or a protein encoded by the gene.

3. The composition of claim 1, wherein the agent for measuring the expression level of the mRNA of the gene is one or more selected from the group consisting of primers, probes, and antisense nucleotides that specifically bind to the mRNA of the gene.

4. The composition of claim 2, wherein the agent for measuring the expression level of the protein is one or more selected from the group consisting of polypeptides, compounds, antibodies, and aptamers that specifically bind to the protein.

5. A kit for diagnosing radiosensitivity of breast cancer, the kit comprising: the composition of any one of claims 1 to 4.

6. The kit of claim 5, wherein the kit is an RT-PCR kit, a microarray chip kit, a DNA kit, an ELISA kit, a protein chip kit, or a rapid kit.

7. A method for providing information for diagnosing radiosensitivity of breast cancer, the method comprising:
(a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes; and
(b) comparing the expression levels of the mRNA or proteins with expression levels of mRNA or proteins of a normal control group.

8. The method of claim 7, wherein (a) comprises measuring an expression level of mRNA of an LCN2 gene, or a protein encoded by the gene.

9. A composition for predicting a prognosis of breast cancer radiotherapy, the composition comprising: an agent for measuring expression levels of mRNA of two or more genes selected from the group consisting of CD68, IFI16, TNFR2, CXCL5, LCN2, SERPING1, MATN1, and SYTL4, or proteins encoded by two or more of the genes.

10. The composition of claim 9, wherein the composition comprises an agent for measuring an expression level of mRNA of an LCN2 gene, or a protein encoded by the gene.

11. The composition of claim 9, wherein the agent for measuring the expression level of the mRNA of the gene is one or more selected from the group consisting of primers, probes, and antisense nucleotides that specifically bind to the mRNA of the gene.

12. The composition of claim 9, wherein the agent for measuring the expression level of the protein is one or more selected from the group consisting of antibodies and aptamers that specifically bind to the protein.

13. A kit for predicting a prognosis of breast cancer radiotherapy, the kit comprising: the composition of any one of claims 9 to 12.

14. The kit of claim 13, wherein the kit is an RT-PCR kit, a microarray chip kit, a DNA kit, an ELISA kit, a protein chip kit, or a rapid kit.

15. A method for providing information for predicting a prognosis of breast cancer radiotherapy, the method comprising:
(a) measuring, in a sample isolated from a subject, expression levels of mRNA of two or more genes selected from the group consisting of CD68, IFI16, TNFR2, CXCL5, LCN2, SERPING1, MATN1, and SYTL4, or proteins encoded by two or more of the genes; and
(b) comparing the expression levels of the mRNA or proteins with expression levels of mRNA or proteins of a normal control group.

16. The method of claim 15, wherein (a) comprises measuring an expression level of mRNA of an LCN2 gene, or a protein encoded by the gene.
